# EUROPÄISCHE PATENTSCHRIFT

(11) **EP 0 866 814 B1**
(45) Veröffentlichungstag und Bekanntmachung des Hinweises auf die Patenterteilung: **22.03.2000**
(21) Anmeldenummer: 96943078.4
(22) Anmeldetag: 11.12.1996
(51) Int. Cl.: C08F 220/04

(54) **COPOLYMERISATE AUS OLEFINISCH UNGESÄTTIGTEN MONOCARBONSÄUREN, OLEFINISCH UNGESÄTTIGTEN DICARBONSÄUREN ODER DEREN ANHYDRIDEN ODER MISCHUNGEN SOLCHER CARBONSÄUREN ODER CARBONSÄURENANHYDRIDEN UND IHRE VERWENDUNG ALS VERDICKUNGS- ODER DISPERGIERMITTEL**
COPOLYMERS OF OLEFINICALLY UNSATURATED MONOCARBOXYLIC ACIDS, OLEFINICALLY UNSATURATED DICARBOXYLIC ACIDS OR THEIR ANHYDRIDES OR MIXTURES OF SUCH CARBOXYLIC ACIDS OR ANHYDRIDES AND THEIR USE AS THICKENERS AND DISPERSANTS
COPOLYMERES D'ACIDES MONOCARBOXYLIQUES A INSATURATION OLEFINIQUE, D'ACIDES DICARBOXYLIQUES A INSATURATION OLEFINIQUE OU LEURS ANHYDRIDES, OU DE MELANGES DE TELS ACIDES CARBOXYLIQUES OU ANHYDRIDES D'ACIDES CARBOXYLIQUES ET LEUR UTILISATION COMME AGENTS EPAISSISSANTS OU DISPERSIFS

(30) Priorität: 14.12.1995 DE 19546698
(43) Veröffentlichungstag der Anmeldung: 30.09.1998
(73) Patentinhaber: BASF AKTIENGESELLSCHAFT, 67056 Ludwigshafen (DE)
(72) Erfinder: SCHADE, Christian, D-67061 Ludwigshafen (DE); WEKEL, Hans-Ulrich, D-67158 Ellerstadt (DE); SANNER, Axel, D-67227 Frankenthal (DE); SPERLING, Karin, D-67433 Neustadt (DE)
(86) Internationale Anmeldenummer: EP9605522
(87) Internationale Veröffentlichungsnummer: WO9721744

(56) Entgegenhaltungen:
- EP-A- 0 335 624
- WO-A-93/22357

## Beschreibung

Die vorliegende Erfindung betrifft neue Copolymerisate aus Carbonsäuren, mehrfach olefinisch ungesättigten Derivaten der allgemeinen Formel I und gegebenenfalls einen oder mehreren copolymerisierbaren Monomeren. Weiterhin betrifft die vorliegende Erfindung die Verwendung dieser Copolymerisate als Verdickungs- oder Dispergiermittel, insbesondere in kosmetischen Zubereitungen, sowie diese Copolymerisate enthaltende pharmazeutische und insbesondere kosmetische Zubereitungen.

In WO 93/22357 wird die Verwendung von Copolymerisaten in kosmetischen oder pharmazeutischen Zubereitungen beschrieben, die durch radikalisch initiierte Polymerisation erhältlich sind. Für die Polymerisation werden folgende Monomere verwendet:
a) olefinisch ungesättigte Monocarbonsäuren, Dicarbonsäuren oder deren Anhydriden oder Mischungen solcher Carbonsäuren oder Carbonsäureanhydriden
b) eine oder mehrere langkettige Verbindungen mit isolierten C-C-Mehrfachbindungen, und ggf.
c) weitere copolymerisierbare Monomere sowie
d) ein oder mehrere Verbindungen mit mindestens zwei olefinisch ungesättigten Gruppen im Molekül als Vernetzer.

Diese Copolymerisate werden als Verdickungs- oder Dispergiermittel verwendet.

Als übliche Verdickungsmittel oder Viskositätsregler werden Copolymerisate aus olefinisch ungesättigten Carbonsäuren wie (Meth)Acrylsäure, Maleinsäure oder Maleinsäureanhydrid, hydrophoben Comonomeren wie Ester der (Meth)Acrylsäure und geringen Mengen eines Vernetzers, d.h. einer Verbindung mit mindestens zwei olefinisch ungesättigten Gruppen im Molekül, eingesetzt. Derartige Copolymerisate sind beispielsweise in der EP-A 328 725 und der EP-A 435 066 beschrieben.

Um eine ausreichende Verdickungswirkung zu erzielen, muß eine Vernetzung während der Polymerisation stattfinden. Mit den üblicherweise eingesetzten Vernetzungskomponenten, beispielsweise Divinylbenzol, Pentaerythrittriallylether, Diallylweinsäurediamid, Bisacrylamidoessigsäure, Methylenbisacrylamid, Methacrylsäureallylester, Trimethylolpropandiallylether oder Allylethern der Saccharose, bringt die Steuerung der Vernetzung aber noch Probleme mit sich. Aufgrund der hohen Doppelbindungsdichte sind viele dieser Verbindungen sehr reaktiv, bilden daher ein zusätzliches Sicherheitsrisiko, sind schwer zu lagern und besitzen ein erhöhtes hautreizendes Potential. Außerdem verläuft dadurch die Vernetzung im allgemeinen sehr heterogen, so daß die Polymerisate noch größere Mengen unvernetzter Anteile enthalten. Aufgrund der hohen Reaktivität der Vernetzer werden im allgemeinen nur geringe Mengen der vernetzenden Komponente benutzt, bereits geringe Schwankungen in der Vernetzerkonzentration, wie sie in Produktionsprozessen vorkommen, führen daher zu deutlichen Schwankungen in der Verdickungswirkung der Copolymerisate.

Aufgabe der vorliegenden Erfindung war es daher, ein Polymerisat, welches als Verdickungs- oder Dispergiermittel eingesetzt werden kann, bereitzustellen, dessen Verdickungswirkung besser gesteuert werden kann.

Demgemäß wurde ein Copolymerisat gefunden, welches erhältlich ist durch radikalisch initiierte Polymerisation von
A) 70 bis 99,9 Gew.-% einer olefinisch ungesättigten C₃- bis C₅-Monocarbonsäure, einer olefinisch ungesättigten C₄- bis C₈-Dicarbonsäure oder ihres Anhydrids oder einer Mischung solcher Carbonsäuren oder Carbonsäureanhydride mit
B) 0,1 bis 30 Gew.-% eines oder mehrerer mehrfach olefinisch ungesättigter Derivate der allgemeinen Formel I 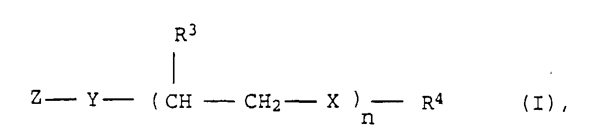 in der
   - Z: für eine Vinyl- oder Allylgruppe oder die Struktur 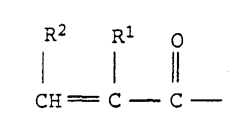 steht und
   - R¹,R²: gleich oder verschieden sind und Wasserstoff oder Methyl bezeichnen,
   - R³: Wasserstoff, Methyl oder Ethyl bezeichnet,
   - R⁴: für einen ein- oder mehrfach olefinisch ungesättigten C₆- bis C₃₀-Alkenyl- oder -Cycloalkenylrest mit 5 bis 8 Kohlenstoffatomen im Cyclus oder für einen ein- oder zweifach olefinisch ungesättigten Arylalkenylrest mit insgesamt 9 bis 15 C-Atomen steht,
   - X: Sauerstoff oder NH bezeichnet,
   - Y: Sauerstoff, NH oder N-Alkyl bezeichnet,
   - n: für eine Zahl von 0 bis 50 bedeuten und
C) 0 bis 29,9 Gew.-% einer oder mehrerer copolymerisierbarer Monomere.

In einer bevorzugten Ausführungsform ist das erfindungsgemäße Copolymerisat aufgebaut aus
A) 80 bis 99,5 Gew.-%, insbesondere 90 bis 99 Gew.-% der Carbonsäure-Komponente A,
B) 0,5 bis 20 Gew.-%, insbesondere 1 bis 10 Gew.-% des mehrfach olefinisch ungesättigten Derivates I und
C) 0 bis 19,5 Gew.-%, insbesondere 0 bis 9 Gew.-% einer oder mehrerer copolymerisierbarer Monomere.

Als Komponente A eignen sich vor allem Acrylsäure, Methacrylsäure oder Maleinsäureanhydrid, daneben aber auch Crotonsäure, 2-Pentensäure, Maleinsäure, Fumarsäure oder Itaconsäure.

Der Rest R¹ in den Derivaten I bezeichnet Wasserstoff oder Methyl.

Der Rest R² in den Derivaten I bezeichnet vorzugsweise Wasserstoff.

Der Rest R³ in den Derivaten I steht für Wasserstoff, Methyl oder Ethyl, bevorzugt für Wasserstoff oder Methyl.

Die Variable X in den Derivaten I steht vorzugsweise für Sauerstoff.

Die Variable Y in den Derivaten I steht für Sauerstoff, NH oder N-Alkyl. Als N-Alkylreste eignen sich bevorzugt C₁-C₄-Alkylreste wie Methyl-, Ethyl-, Propyl-, i-Propyl-, Butyl- i-Butyl und t-Butyl. Besonders bevorzugt steht Y für Sauerstoff und NH, ganz besonders bevorzugt für Sauerstoff.

Der Alkoxylierungs- bzw. Alkiminierungsgrad n beträgt vorzugsweise 0 bis 20, insbesondere 0 bis 7, ganz besonders bevorzugt wird 0 oder 1.

Der Rest R⁴ in den Derivaten I bezeichnet einen ein -oder mehrfach olefinisch ungesättigten C₆-bis C₃₀-Alkenyl -oder Cycloalkenylrest mit 5 bis 8 Kohlenstoffatomen im Cyclus oder einen ein -oder zweifach olefinisch ungesättigten Arylalkylrest bevorzugt Phenylalkenyl -oder Naphtylalkenylrest mit insgesamt 9 bis 15 C-Atomen.

Beispiele für den Rest R⁴ sind die langkettigen oder sterisch anspruchsvollen Reste folgender ungesättigter Alkohole: 2-Hexen-1-ol, 5-Hexen-1-ol, 3-Hexen-1-ol, 1-Octen-3-ol, 9-Decen-1-ol, 13-Tetradecen-1-ol, Citronellol, Nerol, Linalool, Terpineol, Nopol, Farnesol, Linolylalkohol, Linolenylalkohol, 13-Docosendol, 12-Hydroxy-9-octadecanol, Palmitoleylalkohol, Oleylalkohol, 2-Phenyl-2-propen-l-ol (Zimtalkohol) oder 5-Phenyl-2,4-pentadien-1-ol. Hiervon wird der Oleylrest besonders bevorzugt.

Ganz besonders bevorzugt werden als Komponente B mehrfach olefinisch ungesättigte Carbonsäurederivate der allgemeinen Formel Ia 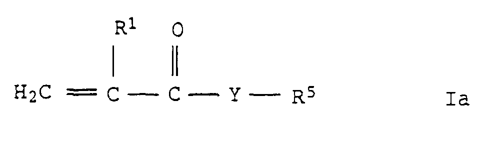 in der R¹ und Y die oben genannten Bedeutungen haben und R⁵ für einen ein- bis dreifach olefinisch ungesättigten C₈- bis C₁₈-Alkenyl- oder -Cycloalkenylrest mit 5 bis 8 Kohlenstoffatomen im Cyclus oder für einen ein- oder zweifach olefinisch ungesättigten Phenylalkenylrest mit insgesamt 9 bis 12 C-Atomen steht.

Die Derivate I bzw. die Carbonsäurederivate Ia sind nach bekannten Herstellungsverfahren leicht zugänglich, beispielsweise durch Veresterung von (Meth)Acrylsäure mit Alkoholen der Formel R⁴/R⁵-OH oder ethoxylierten, propoxylierten oder butoxylierten Alkoholen der Formel R⁴/R⁵-(O-CH₂-CHR²)ₙ-OH oder durch Umsetzung von reaktiven (Meth)Acrylsäurederivaten mit Aminen der Formel R⁴/R⁵⁻NH₂ sowie deren ethoxylierten, propoxylierten oder butoxylierten Derivaten.

Anstelle der (Meth)Acrylester können auch die entsprechenden Allylether, Vinylether oder Vinylester eingesetzt werden. Umsetzungsprodukte reaktiver Monomere wie z.3. Glycidyl(meth)acrylat oder (Meth)Acrylisocyanat mit den beschriebenen Alkoholen und Aminen ergeben ebenfalls geeignete Comonomere B.

Als weitere copolymerisierbare Monomere C zur geringfügigen Modifizierung der erfindungsgemäßen Copolymerisate eignen sich beispielsweise N-Vinylpyrrolidon, N-Vinylcaprolactam, C₁- bis C₃₀-Alkyl(meth)acrylate, z.B. Methyl(meth)acrylat, Ethyl(meth)acrylat, Lauryl(meth)acrylat, Stearyl(meth)acrylat, oder technische Mischungen dieser Verbindungen, (Meth)Acrylamid oder N-(C₁- bis C₁₈-Alkyl)(meth)acrylamide, z.B. N-tert.-Butylacrylamid oder N-tert.-Octylacrylamid, C₈-C₃₀-1-Alkene wie 1-Octen, 1-Dodecen, 1-Tetradecen, 1-Hexadecen, 1-Octadecen, 1-Eicosen, 1-Tetracosen, C₁- bis C₁₈-Vinylester wie Vinylacetat, Vinylpropionat oder Versaticsäurevinylester, (Meth)Acrylester von Ethylenglycol, Diethylenglycol oder ihren höheren Homologen sowie den daraus abgeleiteten Monoalkylethern wie z.B. Diethylenglycolmonoethylether. Selbstverständlich können auch Mischungen der genannten Monomere C eingesetzt werden. Besonders bevorzugte Monomere C sind C₈-C₂₄-Alkyl(meth)acrylate und C₈-C₃₀-Alkene.

In besonderen Fällen können der Reaktionsmischung zur Unterstützung der Vernetzung geringe Mengen einer mehrfach ethylenisch ungesättigten Verbindung zugesetzt werden.

Zur Herstellung der erfindungsgemäßen Copolymerisate können die Monomere A bis C im Prinzip nach allen bekannten Verfahren radikalisch polymerisiert werden. Eine besonders gut geeignete Herstellungsmethode ist die Fällungspolymerisation, bei der die Monomeren, nicht aber das Polymere im eingesetzten Lösungsmittel-System löslich sind.

Als Lösungsmittel sind beispielsweise aromatische und gesättigte aliphatische Kohlenwasserstoffe geeignet. Beispiele für aromatische Kohlenwasserstoffe sind Benzol, Toluol, Xylol und Isopropylbenzol. Die gesättigten aliphatischen Kohlenwasserstoffe haben vorzugsweise 5 bis 12 Kohlenstoffatome. Geeignet sind Pentan, n-Hexan, Cyclohexan, Heptan, Octan und Isooctan. Die Fällungspolymerisation kann auch in halogenierten gesättigten aliphatischen Kohlenwasserstoffen wie 1,1,1-Trichlorethan oder Methylenchlorid durchgeführt werden. Als Reaktionsmedium eignen sich außerdem Ether, C₂-C₆-Alkylester der Ameisensäure oder der Essigsäure, Ketone mit 3 bis 6 Kohlenstoffatomen, flüssiges oder überkritisches Kohlendioxid, Ethan, Propan oder Butan. Geeignete Ether sind beispielsweise tert.-Butylmethylether oder Isobutylmethylether. Die Alkylester der Ameisensäure oder Essigsäure leiten sich vorzugsweise von gesättigten Alkoholen mit 2 bis 6 Kohlenstoffatomen ab, z.B. Ameisensäureethylester, Essigsäuremethylester, Essigsäureethylester, Essigsäure(iso)propylester oder Essigsäure(iso)butylester. Geeignete Ketone sind beispielsweise Aceton und Methylethylketon. Die Verdünnungsmittel können alleine oder in Mischung untereinander eingesetzt werden. Vorzugsweise werden als Verdünnungsmittel bei der Fällungspolymerisation gesättigte aliphatische Kohlenwasserstoffe mit 5 bis 8 C-Atomen im Molekül, die geradkettig oder verzweigt, cyclisch oder dicyclisch sein können, Alkylester der Essigsäure, Methylenchlorid oder überkritisches Kohlendioxid eingesetzt. Besonders bevorzugt wird Cyclohexan als Lösemittel bei der Fällungspolymerisation eingesetzt. Die Menge an Lösungsmittel wird vorteilhaft so gewählt, daß die Reaktionsmischung während der Polymerisation gerührt werden kann. Der Feststoffgehalt der Mischung liegt nach der Polymerisation vorzugsweise in dem Bereich von 10 bis 40 Gew.-%.

Das Molekulargewicht der Copolymerisate kann gewünschtenfalls durch Zugabe von Reglern zur polymerisierenden Mischung erniedrigt werden. Geeignete Regler sind beispielsweise Mercaptoverbindungen wie Dodecylmercaptan, Thioethanol, Thioglykolsäure oder Mercaptopropionsäure. Falls Regler mitverwendet werden, werden sie in Mengen von 0,1 bis 5 Gew.-%, bezogen auf die eingesetzten Monomeren, verwendet.

Die Copolymerisation erfolgt in Gegenwart von Radikale bildenden Polymerisationsinitiatoren. Geeignete Verbindungen dieser Art sind Azo- oder Peroxoverbindungen, z. B. Diacylperoxide wie Dilauroylperoxid, Didecanoylperoxid und Dioctanoylperoxid oder Perester wie tert.-Butylperoctanoat, tert.-Butylperpivalat, tert.-Amylperpivalat oder tert.-Butylperneodecanoat wie Azoverbindungen wie Dimethyl-2,2'-azobis(isobutyrat), 2,2'-Azobis(isobutyronitril), 2,2'-Azobis (2-methylbutyronitril) oder 2,2'-Azobis(2,4-dimethylvaleronitril). Die Initiatoren werden in den bei Fällungspolymerisation üblichen Mengen eingesetzt, z. B. in Mengen von 0,05 bis 5 Gew.-%, bezogen auf die Monomeren. Der Polymerisationsmischung können in geringer Menge Wasser, Alkohole, Schutzkolloide, Emulgatoren oder auch größere Mengen einer Base, z.B. Kaliumcarbonat, zugesetzt werden. Falls bei der Fällungspolymerisation Wasser und/oder Basen mitverwendet werden, dann nur in solchen Mengen, daß die Mischung aller Komponenten vor dem Beginn der Polymerisation noch homogen erscheint.

Die Fällungspolymerisation wird üblicherweise unter einer Inertgasatmosphäre durchgeführt. Die Copolymerisation kann beispielsweise in der Art durchgeführt werden, daß man alle Komponenten, die während der Polymerisation anwesend sind, in einem Polymerisationsgefäß vorlegt, die Reaktion startet und das Reaktionsgemisch gegebenenfalls kühlt, um die Polymerisationstemperatur zu kontrollieren. Man kann jedoch auch so vorgehen, daß man nur einen Teil der zu polymerisierenden Komponenten vorlegt, die Polymerisation startet und den Rest der zu polymerisierenden Mischung je nach Fortschritt der Polymerisation kontinuierlich oder diskontinuierlich zudosiert. Man kann jedoch auch so verfahren, daß man zunächst das Verdünnungsmittel zusammen mit einem Tensid vorlegt und die Monomeren und den Polymerisationsinitiator in separaten Zuläufen in die Vorlage kontinuierlich oder diskontinuierlich einbringt.

Die Temperatur während der Polymerisation liegt im allgemeinen zwischen 40 und 160, vorzugsweise 50 bis 120°C. Sie kann während der Reaktion unterschiedlich gesteuert werden. Die Polymerisation wird vorzugsweise unter Atmosphärendruck durchgeführt, kann jedoch auch unter vermindertem oder erhöhtem Druck vorgenommen werden. Sofern die Polymerisationstemperatur oberhalb des Siedepunkts des inerten Verdünnungsmittels liegt, wird die Polymerisation in druckdichten Apparaturen bei Drücken bis zu 8 bar durchgeführt. Falls Kohlendioxid als inertes Verdünnungsmittel eingesetzt wird, wird die Polymerisation üblicherweise in einem Autoklaven oberhalb der kritischen Temperatur von Kohlendioxid durchgeführt. Die Drücke liegen dann oberhalb von 73 bar.

Der Polymerisationsprozeß wird vorzugsweise so gesteuert, daß das Copolymerisat in Form eines feinteiligen Pulvers anfällt. Die mittlere Teilchengröße des Polymerisatpulvers beträgt 0,1 bis 500, vorzugsweise 0,5 bis 200 µm. Nach der Polymerisation wird das vernetzte Copolymerisat von den übrigen Bestandteilen der Reaktionsmischung abgetrennt, beispielsweise abfiltriert, abdekantiert oder abzentrifugiert. Das so erhaltene Pulver kann gegebenenfalls weiteren geeigneten Trenn-, Wasch-, Trocknungs- oder Mahlverfahren unterworfen werden.

Die erfindungsgemäßen Copolymerisate eignen sich gut zur Verdikkung von Wasser und wäßriger Systeme, wie Pigmentanschlämmungen in Wasser, Flüssigwaschmitteln, wäßrigen Polymerlösungen oder Polymerdispersionen.

Die oben beschriebenen Copolymerisate werden als Stabilisator in Öl-in-Wasser-Emulsionen in Mengen von 0,01 bis 5 Gew.-%, bezogen auf die Emulsionen, verwendet. Sie eignen sich zum Stabilisieren sämtlicher Öl-in-Wasser-Emulsionen, z. B. von Wasser-in-Öl-Polymeremulsionen, Entschäumern auf Basis von Öl-in-Wasser-Emulsionen, Druckpasten für den Textildruck, Anstrichfarben, Reinigungsmittelformulierungen, Bohrlochspülungen, Flüssigwaschmitteln und insbesondere zur Stabilisierung von kosmetischen oder pharmazeutischen Zubereitungen auf Basis von Öl-in-Wasser-Emulsionen.

Um eine dauerhafte Verdickung von Wasser und wäßrigen Systemen und/oder eine Stabilisation von Öl-in-Wasser-Emulsionen zu erzielen, wird das dispergierte Polymer mit einer Base ausreichend neutralisiert. Geeignete Basen sind z.B. Alkalibasen wie Alkalimetallhydroxide, -hydrogencarbonate und -carbonate, beispielsweise NaOH, KOH und Natrium- und Kaliumcarbonat, Ammoniak und organische Amine, Pyridine und Amidine oder Mischungen daraus. Bei der Neutralisation mit Hilfe organischer Amine verwendet man vorzugsweise Alkanolamine aus der Reihe der Mono- Di- oder Trialkanolamine mit 2 bis 5 C-Atomen im Alkanolrest wie Mono-, Di- oder Triethanolamin, Mono-, Di- oder Tri(iso)propanolamin oder 2-Amino-2-methylpropanol, Alkandiolamine mit 2 bis 4 C-Atomen im Alkandiolrest wie 2-Amino-2-methyl-1,3-propandiol oder 2-Amino-2-ethyl-1,3-propandiol, Alkanpolyolamine wie Tetrahydroxypropylethylendiamin, Tris(hydroxymethyl)aminomethan oder N,N,N',N'-Tetrakis(2-hydroxypropyl)ethylendiamin, Alkylamine wie Di(2-ethylhexyl)amin, Triamylamin oder Dodecylamin und Aminoether wie Morpholin.

Die kosmetischen oder pharmazeutischen Zubereitungen können dabei als Öl alle hierfür üblicherweise verwendbaren Öle enthalten. Die Gesamtmenge der Ölphase in der Emulsion kann dabei bis zu 80 Gew.-% betragen. Der Anteil der Ölphase in den kosmetischen oder pharmazeutischen Zubereitungen liegt bevorzugt bei 10 bis 50 Gew.-%. Die schwach vernetzten Copolymerisate werden vorzugsweise zum Stabilisieren in Cremes oder Lotionen angewandt. Daneben eignen sie sich gut zum Verdicken wäßriger Systeme oder Ausbildung verdickter Gele, nachdem das dispergierte Copolymerisat durch Zugabe einer Base, ausreichend neutralisiert worden ist.

Im Gegensatz zu schwach vernetzten Homopolymerisaten der Acrylsäure gelingt es, mit den erfindungsgemäß zu verwendenden Copolymerisaten Öl-in-Wasser-Emulsionen dauerhaft zu stabilisieren. Die bevorzugt zum Einsatz gelangenden Mengen an vernetzten Copolymerisaten, die durch Fällungspolymerisation hergestellt werden, beträgt 0,05 bis 2 Gew.-%, bezogen auf die Emulsionen.

Durch Einsatz der mehrfach olefinisch ungesättigten Derivate I, welche gleichzeitig einen zur Modifizierung der Carbonsäuren A notwendigen hydrophoben Molekül teil und zur Erzielung der Vernetzereigenschaften mehrere olefinisch ungesättigte Gruppen enthalten, ist die Verwendung der sonst üblichen Vernetzerkomponenten, die eingangs in ihrer Problematik geschildert wurden, weitgehend überflüssig geworden.

Überraschenderweise zeigte das erfindungsgemäße Copolymerisat außerdem verbesserte Anwendungseigenschaften.

Die Viskosität der gefundenen Copolymerisate ist weniger empfindlich gegenüber Schwankungen der eingesetzten Vernetzerkonzentration. Dadurch ist auch bei geringeren Vernetzermengen oder einer uneinheitlichen Copolymerisation eine gleichmäßige Gelviskosität und damit eine konstantere Verdickungswirkung gewährleistet.

### Beispiele

Wenn nichts anderes angegeben ist, beziehen sich die Prozentangaben auf das Gewicht. Die Viskosität wurde, wenn nicht anders beschrieben mit einem Handviskosimeter (Hake VT-02) bei 23°C gemessen.

### Beispiel 1

In einem 3 l-Planschliffkolben wurden 1400 ml 1,1,1-Trichlorethan, 250 g Acrylsäure und 10 g Oleylmethacrylat verrührt und 30 min mit Stickstoff gespült. Im Stickstoff-Strom wurde auf 80°C unter Rühren erwärmt und nach Erreichen dieser Temperatur während 3 h wurden 100 ml 1,1,1-Trichlorethan und 0,4 g Dilauroylperoxid zudosiert. Nach weiteren 3 h wurde abgekühlt, das ausgefallene Produkt abfiltriert, mit 500 ml 1,1,1-Trichlorethan gewaschen und im Vakuum bei 60°C getrocknet.

Zur Bestimmung der Gelviskosität wurden 1,0 g des Polymeren in einem Becherglas in 190 ml Wasser dispergiert. Unter Rühren wurden 10 ml einer 10%igen Triethanolamin-Lösung zugegeben. Die Viskosität des erhaltenen Gels wurde mit einem Handviskosimeter (Hake VT-02) zu 10,0 Pa.s bei 23°C bestimmt. Durch Ausstreichen auf eine Glasplatte konnte man erkennen, daß das Gel glatt und annähernd stippenfrei war.

Zur Kontrolle der Emulgierfähigkeit wurden 0,4 g des Polymeren in ein Becherglas eingewogen und in 30 ml Paraffinöl dispergiert. Danach wurden 100 ml Wasser und anschließend 4 ml einer 10%igen Triethanolamin-Lösung unter intensivem Rühren zugegeben. Die Emulsion wurde mit einem Dispergieraggregat bei 8000 U/min für wenige Sekunden homogenisiert. Die Viskosität wurde wie oben zu 7,5 Pa·s bestimmt. Die Struktur der Emulsion wurden nach 1 h durch Ausstreichen auf eine Glasplatte begutachtet. Zur Bestimmung der Langzeitstabilität wurde die Emulsion in einen 100 ml-Standzylinder gefüllt und nach 14 Tagen bewertet. Die Emulsion zeigte zu diesem Zeitpunkt keine Tendenz, sich zu trennen.

### Beispiel 2

In einem 3000 ml fassenden Kolben, der mit einem Rührer und einer Apparatur für das Arbeiten unter Schutzgas ausgestattet war, wurden 1320 ml Cyclohexan, 50 g Acrylsäure, 1 g Oleylallylether und 80 mg 2,2'-Azo-bis(2-methyl-butyronitril) vorgelegt und unter Rühren im Stickstoff-Strom auf 80°C erhitzt. Nach dem Erreichen dieser Temperatur wurde ein Zulauf aus 200 g Acrylsäure und 4 g Oleylallylether während 2 Stunden und ein weiterer Zulauf aus 80 ml Cyclohexan und 320 mg 2,2'-Azobis(2-methyl-butyronitril) während 3 Stunden zugetropft. Nach Beendigung der Zugabe des Polymerisationsinitiators wurde das Reaktionsgemisch noch 3 Stunden bei 80°C gerührt. Das Produkt wurde anschließend über eine Filternutsche abgesaugt und im Vakuum bei 50°C getrocknet. Man erhielt 251 g eines weißen Polymerpulvers mit einer Gelviskosität von 7,5 Pa·s.

### Beispiele 3 bis 7

In Analogie hierzu wurden unter Verwendung verschiedener Mengen an Comonomer B und der gleichen Mengen an Acrylsäure, Solvens und Initiator die Beispiele 3 bis 7 durchgeführt. Die Tabelle I zeigt die Ergebnisse.

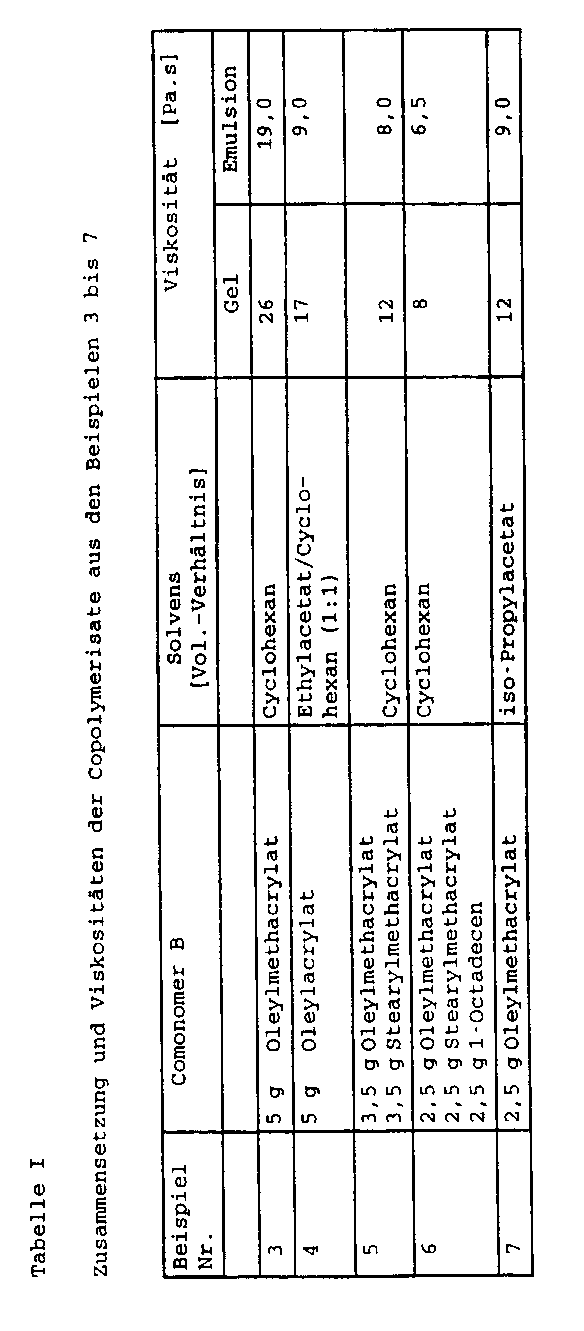

### Beispiele 8 (Vergleichsbeispiel) und 9

In Analogie zu Beispiel 1 wurden unter Verwendung verschiedener Mengen Comonomer B sowie verschiedener Mengen an Pentaerythrittriallylether als Vergleichsvernetzer und der gleichen Menge an Acrylsäure, Cyclohexan als Solvens und Initiator die Beispiele 8 und 9 durchgeführt. Die Tabelle II zeigt die Ergebnisse.

**Tabelle II**

| Vergleichsversuch zwischen Standardvernetzer Pentaerythrittriallylether und Oleylmethacrylat | | | |
|---|---|---|---|
| Beispiel | Vernetzer | Viskosität [Pa·s] | |
| | | Gel | Emulsion |
| 8 a) | 1 g Pentaerythrittriallylether | 8 | 6 |
| b) | 1,5 g Pentaerythrittriallylether | 14 | 9 |
| c) | 2 g Pentaerythrittriallylether | 22 | 16,5 |
| d) | 3 g Pentaerythrittriallylether | 13 | 7,5 |
| 9 a) | 3,75 g Oleylmethacrylat | 17 | 10 |
| b) | 6,25 g Oleylmethacrylat | 27 | 19,5 |
| c) | 7,5 g Oleylmethacrylat | 23 | 17 |
| d) | 8,75 g Oleylmethacrylat | 17 | 10,5 |

Die Ergebnisse zeigen, daß die Gel- und Emulsionsviskosität bei der Verwendung von Oleylmethacrylat weniger empfindlich auf die Vernetzerkonzentration (siehe Grammangaben in Tabelle II) reagiert, als bei der Verwendung von Pentaerythrittriallylether.

## Patentansprüche

1. Copolymerisat, erhältlich durch radikalisch initiierte Polymerisation von
A) 70 bis 99,9 Gew.-% einer olefinisch ungesättigten C₃- bis C₅-Monocarbonsäure, einer olefinisch ungesättigten C₄- bis C₈-Dicarbonsäure oder ihres Anhydrids oder einer Mischung solcher Carbonsäuren oder Carbonsäureanhydride mit
B) 0,1 bis 30 Gew.-% eines oder mehrerer mehrfach olefinisch ungesättigter Derivate der allgemeinen Formel 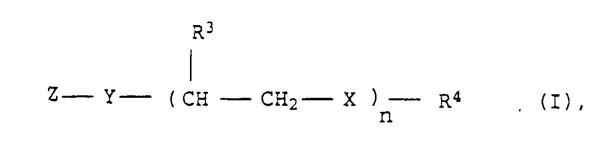 in der
Z für eine Vinyl- oder Allylgruppe oder die Struktur 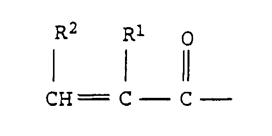 steht und
R¹,R² gleich oder verschieden sind und Wasserstoff oder Methyl bezeichnen,
R³ Wasserstoff, Methyl oder Ethyl bezeichnet,
R⁴ für einen ein- oder mehrfach olefinisch ungesättigten C₆- bis C₃₀-Alkenyl- oder -Cycloalkenylrest mit 5 bis 8 Kohlenstoffatomen im Cyclus oder für einen ein- oder zweifach olefinisch ungesättigten Arylalkenylrest mit insgesamt 9 bis 15 C-Atomen steht,
X Sauerstoff oder NH bezeichnet,
Y Sauerstoff, NH oder N-Alkyl bezeichnet,
n für eine Zahl von 0 bis 50 bedeuten und
C) 0 bis 29,9 Gew.-% einer oder mehrerer copolymerisierbarer Monomere.

2. Copolymerisat nach Anspruch 1, erhältlich durch radikalisch initiierte Polymerisation von
A) 80 bis 99,5 Gew.-% der Carbonsäure-Komponente A,
B) 0,5 bis 20 Gew.-% des mehrfach olefinisch ungesättigten Derivates I und
C) 0 bis 19,5 Gew.-% einer oder mehrerer copolymerisierbarer Monomere.

3. Copolymerisat nach Anspruch 1 oder 2, bei dessen Herstellung als Komponente A Acrylsäure, Methacrylsäure oder Maleinsäureanhydrid verwendet wurden.

4. Copolymerisat nach den Ansprüchen 1 bis 3, bei dessen Herstellung als Komponente B ein oder mehrere mehrfach olefinisch ungesättigte Carbonsäurederivate der allgemeinen Formel Ia 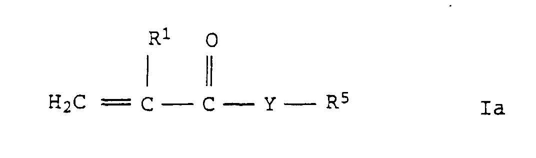 in der R¹ Wasserstoff oder Methyl bezeichnet, Y für Sauerstoff, NH oder N-Alkyl steht und R⁵ für einen ein- bis dreifach olefinisch ungesättigten C₈- bis C₁₈-Alkenyl- oder -Cycloalkenylrest mit 5 bis 8 Kohlenstoffatomen im Cyclus oder für einen ein- oder zweifach olefinisch ungesättigten Phenylalkenylrest mit insgesamt 9 bis 12 C-Atomen steht, verwendet wurden.

5. Verfahren zur Herstellung der Copolymerisate nach einem der Ansprüche 1 bis 4, dadurch gekennzeichnet, daß man die Monomeren radikalisch initiiert polymerisiert.

6. Verwendung von Copolymerisaten gemäß den Ansprüchen 1 bis 4 als Verdickungs- oder Dispergiermittel in wäßrigen Systemen. Kosmetische und pharmazeutische Zubereitungen, enthaltend Copolymerisate gemäß den Ansprüchen 1 bis 4 als Verdikkungs- oder Dispergiermittel in Mengen von 0,01 bis 5 Gew.-%, bezogen auf die Emulsion.

## Claims

1. A copolymer obtainable by free-radical polymerization of
A) 70 - 99.9% by weight of an olefinically unsaturated C₃-C₅-monocarboxylic acid, of an olefinically unsaturated C₄-C₈-dicarboxylic acid or of its anhydride or of a mixture of such carboxylic acids or carboxylic anhydrides with
B) 0.1 - 30% by weight of one or more polyolefinically unsaturated derivatives of the general formula 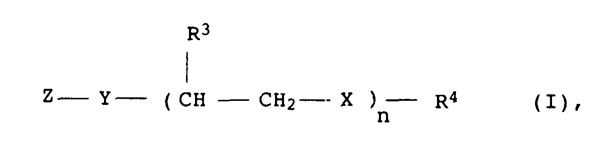 where
Z is a vinyl or allyl group or the structure 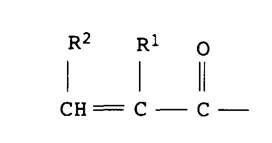 and
R¹, R² are identical or different and are hydrogen or methyl,
R³ is hydrogen, methyl or ethyl,
R⁴ is a mono- or polyolefinically unsaturated C₆-C₃₀-alkenyl or -cycloalkenyl radical having 5 to 8 carbon atoms in the ring or a mono- or diolefinically unsaturated arylalkenyl radical with a total of 9 to 15 carbon atoms,
X is oxygen or NH,
Y is oxygen, NH or N-alkyl,
n is a number from 0 to 50, and
C) 0 - 29.9% by weight of one or more copolymerizable monomers.

2. A copolymer as claimed in claim 1, obtainable by free-radical polymerization of
A) 80 - 99.5% by weight of the carboxylic acid component A,
B) 0.5 - 20% by weight of the polyolefinically unsaturated derivative I and
C) 0 - 19.5% by weight of one or more copolymerizable monomers.

3. A copolymer as claimed in claim 1 or 2, which has been prepared using acrylic acid, methacrylic acid or maleic anhydride as component A.

4. A copolymer as claimed in claims 1 to 3, which has been prepared using as component B one or more polyolefinically unsaturated carboxylic acid derivatives of the general formula Ia 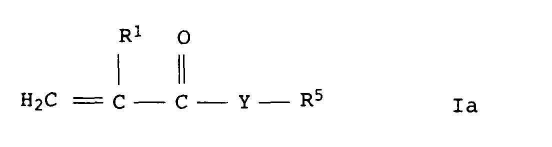 where R¹ is hydrogen or methyl, Y is oxygen, NH or N-alkyl, and R⁵ is a mono- to triolefinically unsaturated C₈-C₁₈-alkenyl or -cycloalkenyl radical having 5 to 8 carbon atoms in the ring or a mono- or diolefinically unsaturated phenylalkenyl radical with a total of 9 to 12 carbon atoms.

5. A process for preparing the copolymers as claimed in any of claims 1 to 4, which comprises free-radical polymerization of the monomers.

6. The use of copolymers as claimed in claims 1 to 4 as thickeners or dispersants in aqueous systems.

7. A cosmetic or pharmaceutical preparation comprising copolymers as claimed in claims 1 to 4 as thickeners or dispersants in amounts of from 0.01 to 5% by weight, based on the emulsion.

## Revendications

1. Copolymère obtenu par polymérisation radicalaire de
**A**) 70 à 99,9 % en poids d'un acide monocarboxylique à insaturation oléfinique en C3-C5, d'un acide dicarboxylique à insaturation oléfinique en C4-C8 ou de son anhydride, ou d'un mélange de tels acides carboxyliques ou anhydrides d'acides carboxyliques, avec
**B**) 0,1 à 30 % en poids d'un ou plusieurs dérivés à insaturation multi-oléfinique de formule générale 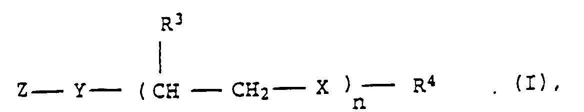 dans laquelle
Z représente un groupe vinyle ou allyle ou la structure 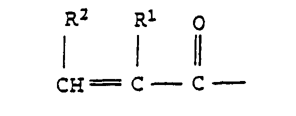 et
R1, R2, ayant des significations identiques ou différentes, représentent chacun l'hydrogène ou un groupe méthyle,
R3 représente l'hydrogène, un groupe méthyle ou éthyle,
R4 représente un radical alcényle en C6-C30 ou cycloalcényle en C6-C30 contenant 5 à 8 atomes de carbone cycliques, à insaturation mono- ou multi-oléfinique, ou un groupe arylalcényle à insaturation mono- ou di-oléfinique, contenant au total 9 à 15 atomes de carbone,
X représente l'oxygène ou NH,
Y représente l'oxygène, NH ou N-alkyle,
n est un nombre allant de 0 à 50, et
**C**) 0 à 29,9 % d'un ou plusieurs monomères copolymérisables.

2. Copolymère selon revendication 1, obtenu par polymérisation radicalaire de
**A**) 80 à 99,5 % en poids du composant acide carboxylique A),
**B**) 0,5 à 20 % en poids du dérivé à insaturation multi-olérinique I et
**C**) 0 à 19,5 % en poids d'un ou plusieurs monomères copolymérisables.

3. Copolymère selon la revendication 1 ou 2, à la préparation duquel on a utilisé en tant que composant A) l'acide acrylique, l'acide méthacrylique ou l'anhydride maléfique.

4. Copolymère selon les revendications 1 à 3, à la préparation duquel on a utilisé en tant que composant B) un ou plusieurs dérivés d'acides carboxyliques à insaturation multi-oléfinique répondant à la formule générale Ia 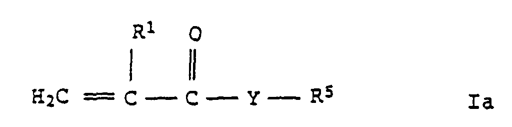 dans laquelle R1 représente l'hydrogène ou un groupe méthyle, Y représente l'oxygène, NH ou N-alkyle et R5 représente un groupe alcényle ou cycloalcényle à insaturation mono- à tri-oléfinique contenant 5 à 8 atomes de carbone cycliques, ou un groupe phénylalcényle à insaturation mono- ou di-oléfinique contenant au total 9 à 12 atomes de carbone.

5. Procédé de préparation des copolymères selon l'une des revendications 1 à 4, caractérisé par le fait que l'on soumet les monomères à polymérisation radicalaire.

6. Utilisation des copolymères selon les revendications 1 à 4 en tant qu'agents épaississants ou dispersants dans des milieux aqueux.

7. Compositions cosmétiques et pharmaceutiques contenant des copolymères selon les revendications 1 à 4 en tant qu'agents épaississants ou dispersants en quantités de 0,01 à 5% du poids de l'émulsion.
